# EUROPEAN PATENT APPLICATION

(11) **EP 2 762 137 A1**
(43) Date of publication of application: **06.08.2014**
(21) Application number: 13191261.0
(22) Date of filing: 01.11.2013
(51) Int. Cl.: A61K 31/366, A61P 19/00, A61P 25/28, A61P 11/00

(54) **Use of myricetin or derivatives thereof as a cathepsin k inhibitor**

(30) Priority: 05.02.2013 CN 201310046002
(71) Applicant: Jilinsheng Jinziyuan Biotechnology Ltd., 136001 Siping Jilin (CN)
(72) Inventor: Deng, XiaoXia, 100085 Beijing (CN); Pang, Yu, 134000 Tonghua City (CN); Liu, Shuang, 130022 Changchun (CN)
(74) Representative: Bond, Christopher William

(57) **Abstract**

This invention is directed to a novel inhibitor on collagen degradation caused by the activity of a human cathepsin K rising. In particular, the inhibitor is myricetin or derivatives thereof. The invention also relates to use of the novel inhibitor to manufacture a medicament in a form of pharmaceuticals, foods, nutraceuticals or cosmetics for the prevention, amelioration and/or treatment of pathological symptoms or diseases associated with collagen degradation. Alternatively, the present invention is further directed to addition of the novel inhibitor to a medicament, food, nutraceutical or cosmetics used for the treatment, amelioration and/or prevention of the disease or pathological symptoms associated with collagen degradation by the increased human cathepsin K activety. The invention also provide the methods for treating, ameliorating and/or preventing the symptoms or diseases, comprising administering an effective amount of the medicament to a subject in need thereof.

## Description

### FIELD OF THE INVENTION

The invention relates to novel inhibitors that inhibit collagen degradation caused by the increased activity of human Cathepsin K; in particular, the inhibitors are myricetin or derivatives thereof. In addition, the present invention is also directed to the novel inhibitor used in manufacturing a pharmaceutical drug, food, nutraceutical or cosmetics, and to compositions thereof for treatment, amelioration and/or prevention of disease or pathological symptoms related to collagen degradation due to the increased cathepsin K. The invention also relates to the methods for treating, ameliorating and/or preventing the symptoms or diseases, comprising administering an effective amount of the medicament to a subject in need thereof.

### BACKGROUND OF THE PRESENT INVENTION

Cathepsin K is a family member of cysteine proteases. It exists in a variety of tissue cells and participates in the catabolism of connective tissue. Comparing to other family members, cathepsin K has the highest collagenase activity, is able to dissociate the triple helix structure of collagen at multiple sites and thus degrade the collagen (Barry ZT, Platt MO. Cathepsin S cannibalism of cathepsin K as a mechanism to reduce type I collagen degradation. J Biol Chem. 2012 Jun 22; Linnevers CJ, McGrath ME, Armstrong R, Mistry FR, Barnes MG, Klaus JL, Palmer JT, Katz BA, Brömme D. Expression of human cathepsin K in Pichia pastoris and preliminary crystallographic studies of an inhibitor complex. Protein Sci. 1997 Apr;6(4):919-21.). Collagen mainly exists in the connective tissue of mammals, and is very important for skin, blood vessels, bones,tendons, teeths and cartilage formation in animal and human. It is the major material basis of the connective tissue. In another word, collagen is the life bracket of animal and human. To the knowledge of the inventors, there is no report on the relationship between the compounds such as myricetin or its derivatives and cathepsin activity, especially the effects of the compounds on the prevention, amelioration and/or treatment of pathological symptoms or diseases caused by collagen degradation due to the human cathepsin K activity rising, or using them to manufacture a medicament contained in a form of pharmaceuticals, foods, nutraceutical or cosmetics.

### SUMMARY OF THE PRESENT INVENTION

The aim of the present invention is to provide a novel cathepsin K inhibitor, in particular, which is myricetin or derivatives thereof, effectively preventing and/or treating pathological symptoms or diseases caused by collagen degradation due to the increased activity of human cathepsin K.

The present invention can be carried out as follows: a new medicament, comprising myricetin or derivatives thereof, preventing and/or treating pathological symptoms or diseases caused by collagen degradation due to the inhibition of human cathepsin K activity is provided.

The pathological symptoms or the diseases comprising one or more the symptoms or the diseases selected from the group consisting of arthritis, rheumatoid spondylitis, multiple myeloma, disc injury, osteoporosis, cardiovascular disease, myocarditis, deposition of lipids caused by high cholesterol, high blood pressure, metabolic disorders, obesity, atherosclerosis, prostatitis, respiratory infectious diseases, Alzheimer's disease, diabetes, aging disease, coronary heart disease, male sexual function, loss of appetite, chronic obstructive pulmonary emphysema, hepatitis, fatigue, neurasthenia, cerebral infarction, asthma, cough, adult respiratory distress syndrome and the like. Preferably, the pathological symptoms or the disease is arthritis, or rheumatoid spondylitis, or the disease is arthritis, rheumatoid spondylitis.

The provided are myricetin or derivatives thereof represented by formula (I) and (II), comprising isomers, racemic or non-racemic mixtures of the isomers, oxides and pharmaceutically acceptable salts and hydrates thereof, for use in therapy and/or manufacture of a medicament for preventing, ameliorating and/or treating pathological symptoms or diseases caused by collagen degradation due to the increased activity of human cathepsin K, 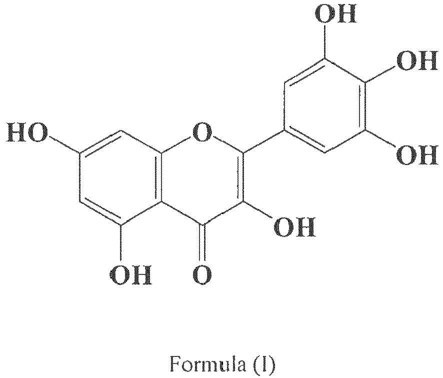 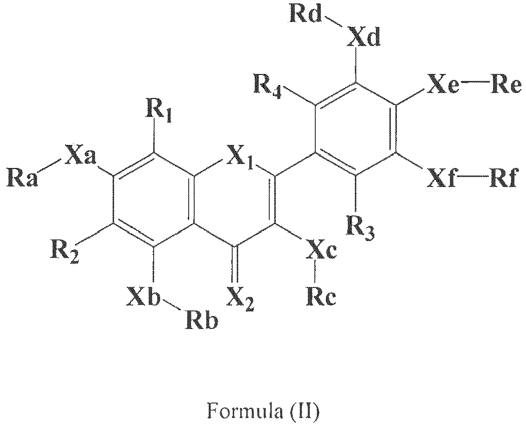 wherein
Xa, Xb, Xc, Xd, Xe, Xf, X₁ and X₂ is each independently selected from oxygen atom (O), sulfur atom (S); Ra, Rb, Rc, Rd, Re and Rf is each independently selected from hydrogen atom (H), (C₁-C₁₀) or (C₁-C₁₀)-O-(C₁-C₁₀) alkyl substituted by 1 to 5 Ry, Ry is selected from Rq, or (C₂ -C₁₀) alkyl, (C₃ -C₁₀) akyl, (C₃ -C₁₀) cycloalkylalkyl, (C₈-C₁₄) dicycloalkyl, (C₈-C₁₄)tricycloalkylalkyl, (C₅-C₁₀) cycloalkylakyl, (C₃ -C₁₀) cycloalkyl, (C₈-C₁₄) dicycloalkylalkyl, (C₈-C₁₄) tricycloalkylalkyl, phenyl, naphthyl, (C₁₄) aryl, each of them can be substituted by one or more Rz, Rz is (C₁-C₆) alkyl, (C₂ -C₆) alkenyl, (C₁ -C₆) alkynyl, (C₃ -C₈) cycloalkyl, (C₆-C₈) cycloalkenyl, phenyl, 3-5 membered heterocyclics, C(halo) ₃ , CH(halo) ₂;
R₁, R₂, R₃ and R₄ is each independently selected from hydrogen atom (H), (C₁-C₁₀) alkyl, (C₂-C₁₀) alkenyl, (C₂-C₁₀) alkynyl, (C₃-C₁₀) cycloalkyl, (C₈-C₁₄) dicycloalkyl, (C₈-C₁₄) tricycloalkylalkyl, phenyl, naphthyl, (C₁₄) aryl, each of them can be non-substituted, or one or more substituted Rq with one or more Rz, Rq can be CN, OH, halogen, N₃, NO₂, N(Rz)₂, =NRz, CH=NRz, NRzOH, ORz, CORz, C(O)ORz, OC(O)Rz, OC(O)ORz, SRz, S(O)Rz or S(O)₂Rz.
Preferably, Xa, Xb, Xb, Xd, Xe, Xf, X1 and X2 is each independently selected from oxygen atom (O). Preferably, Ra, Rb, Rc , Rd, Re, Rf, R1, R2, R3 and R4 is each independently selected from hydrogen atom (H).

In another aspect of the present invention, manufacturing a pharmaceutical drug comprising the compound represented by formula (I) or formula (II) in a form of oral formulation, topical formulation, inhalation formulation, nasal spray, rectal preparations, percutaneous preparations and injection preparation. Preferably, is in a form of oral formulation, topical formulation or inhalation formulation.

Also, the novel inhibitor is used in manufacturing a medicament, food, nutraceutical or cosmetics, and to compositions thereof for treatment, amelioration and/or prevention of disease or pathological symptoms related to collagen degradation due to the increased cathepsin K activity.

Also the disclosure herein generally directed method of treating, preventing and/or ameliorating pathological symptoms or diseases caused by collagen degradation due to the increased activity of human cathepsin K, for example, provided herein is a method, comprising administering an effective amount of a medicament in a subject in need thereof.

Preferably, the subject is human suffering from, or being at the risk of the symptoms or disease, and the effective amount comprising administering ranging from about 0.1 mg/kg to about 1000 mg/kg body weight per day to a patient in need thereof. Such compositions may be for example administered orally, topically or inhalatively.

In some aspects of this invention, disclosed medicament of merycetin or its delivertives may further comprising the other compounds, herbs, a pharmaceutically acceptable excipient, and/or carrier for treating, ameliorating and/or preventing the pathological symptoms or disease. The amount of myricetin or its derivatives is about 10%-80% by weight of the entire pharmaceutical composition.

The present invention is further directed to the addition of the compound presented by formula (I) or (II), or the pharmaceutically acceptable salts thereof to food, nutraceutical or cosmetics used for the treatment, amelioration and/or prevention of the disease or pathological symptoms associated with collagen degradation by the increased human cathepsin K activety.

Surprisingly, the inventors discovered that the cathepsin K inhibitor, myricetin and its derivatives extracted and purified from herbs or chemically synthesized did not show any side effects on human, the medicament comprising one of them can effectively treat, ameliorate and/or prevent the symptoms or disease such as osteoporosis, arthritis, rheumatoid spondylitis, disc injury, myocarditis, and metabolic disorders caused by the deposition of lipids, obesity, hyperlipidemia, arteriosclerosis, and hypertension caused by collagen degradation due to the increased cathepsin K activity. Also, the present invention provide a new mechanism and function of myricetin or derivatives thereof which will contribute to scientific use of the medication, easy to find effective targeted medicament, receiving a significant effect and benefit to human health.

### Figure Description

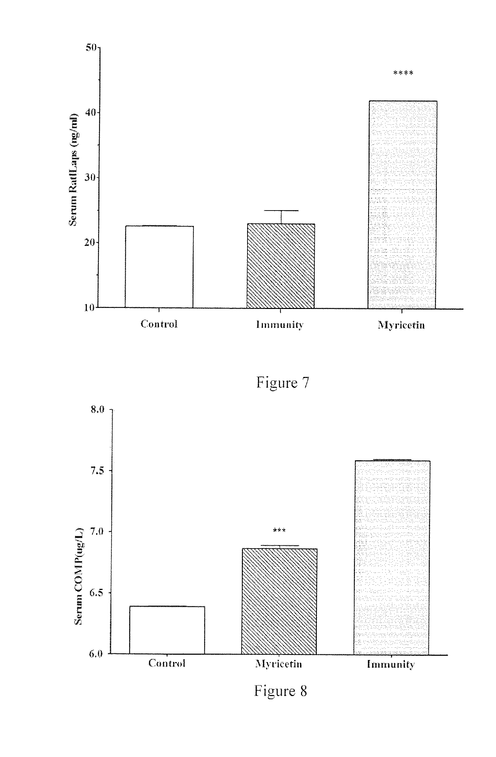

Figure 1. Effect of myricetin on cathepsin K activity.
Figure 2. The effect of myricetin at different concentrations on cathepsin K activity.
   Using a Fluoroskan Ascent, recombinant cathepsin K inhibition ratios with different concentration of myricetin were calculated after completion of the reaction and the IC₅₀ value was determined.
Figure 3. The thickness of right hind paw of bovine type II collagen-induced arthritis mice.
   Myricetin was dosed (with a daily dose of 25 mg / kg) from days 24 to 48 post-immunization. The thickness of the right hind paw in all mice were measured using micrometer calipers, all data are expressed as mean ± SEM.
Figure 4. Body weight change of bovine type II collagen-induced arthritis mice after myricetin treatment. The mice from all three groups were weighed using an electronic balance prior to the first immunization and then every three days post-immunization. All data are expressed as mean ± SEM.
Figure 5. The effects of myricetin on clinical severity of collagen-induced arthritis.
   Myricetin was dosed (25 mg/kg p.o.) from days 24 to 48 post-immunization. Disease severity was assessed using a clinical score, which was graded on a scale of 0-3 per paw (maximum score 12 per mouse). The clinical scores of diseased mice were compared between the myricetin and immune control groups after therapeutic dosing. **p*<0.05 and ***p*<0.01.

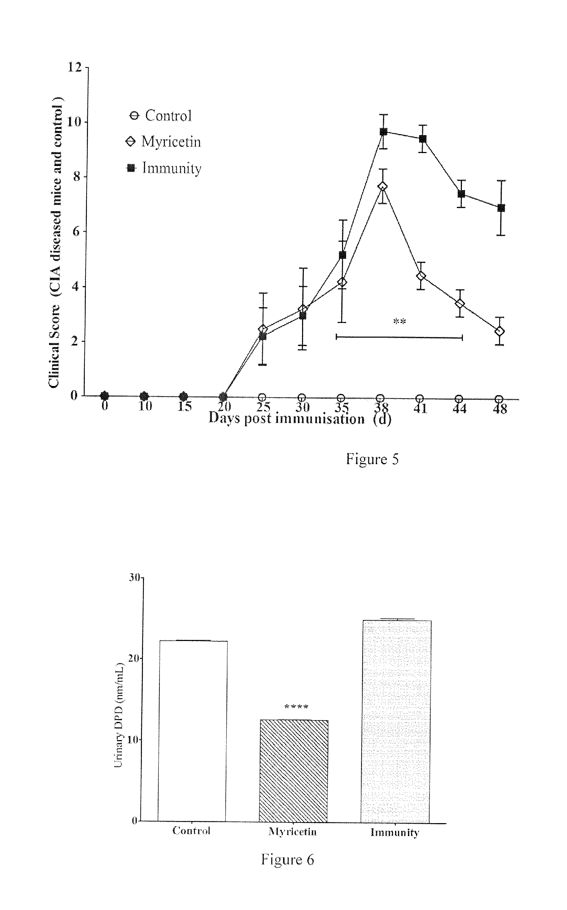

Figure 6-8. Analysis of cartilage and bone degradation markers.
   After therapeutic dosing for 48 days, urinary deoxypyridinoline (DPD) (Figure 6), serum RatLaps (Figure 7) and serum cartilage oligomeric matrix protein (COMP) (Figure 8) were measured by ELISA at the end of the study. Values shown are mean ± SEM. For the control group (n = 10, empty), myricetin at 25 mg/kg (n = 10, diagonal lines), immunized group (n = 10, dots). Student's t-test was used when comparing the myrisetin-treated mice group with the immunized mice group. **p*<0.05, ****p*<0.005, *****p*<0.001.

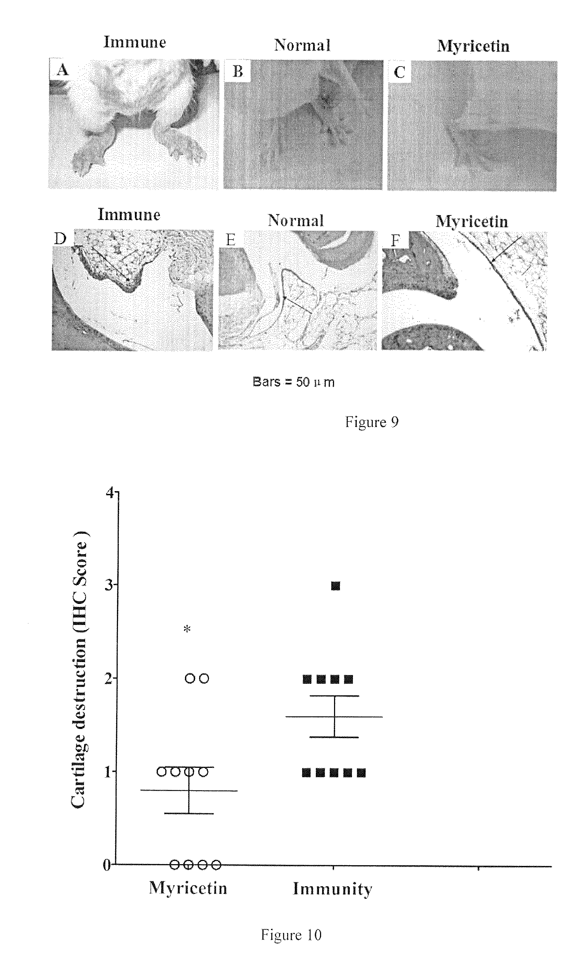

Fig 9. The appearance of the mouse and pathological section.

At the end of study, representative photographs of hind leg from the immune group revealed marked swelling and redness with ankle joint thickening (A), that was attenuated following treatment with myricetin (B).The appearance of normal hind leg is presented in C. Histopathologically, hematoxylin and eosin staining of the ankle joints sctions of immune mice (D) exhibited mild focal hyperplasia of the synovial lining cells (arrow head), that was undetectable in mice treated with myricetin (E) (arrow) and in normal mice (F) (arrow). Scale bar = 50µm.

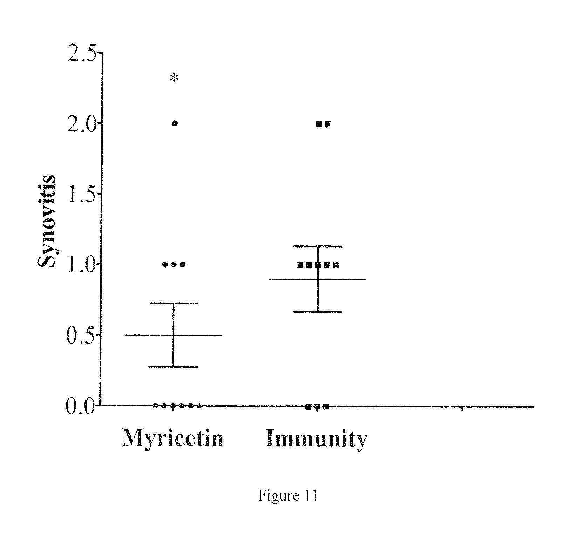

Figure 10-11. Histopathological effects of myricetin (25 mg/kg, p.o.) on cartilage. Hind paws were collected at the end of the therapeutic dosing study, embedded in paraffin, sectioned and stained with hematoxylin/eosin for evaluation of histological parameters or cartilage destruction. Each parameter was scored on a scale of 0-3, with two paw cavities per paw being evaluated and n = 10 per group for the therapeutic dosing study and the immune group (Fig. 10). The Mann-Whitney U test was used for comparisons between the immune group (open squares) and the myricetin-treated group (open circles). In addition, synovitis and synovial fluid (Fig. 11) were measured. Each parameter was scored on a scale of 0-3, n= 10 per group for the therapeutic dosing study and the immune control group. All data are expressed as mean ± SEM. **p*<0.05, ***p*<0.01.

### Detailed Description

It is to be appreciated that a certain aspects, modes, embodiments, variations and features of the invention are described below in various levels of detail in order to provide a substantial understanding of the present invention.

In practicing the present invention, many conventional techniques such as molecular biology, cellular biology,protein biochemistry, immunology, microbiology, recombinant DNA, organic compound synthesis are used. These techniques are well-known and are explained in, e.g. , Sambrook et al., Molecular Cloning: A Laboratory Manual, Second Ed. (Cold Spring Harbor Laboratory Press, Cold Spring Harbor, NY, 1989); DNA Cloning: A Practical Approach, Vols. 1 and II, Glover, Ed.(1985); Oligonucleotide synthesis, Gait, Ed. (1984); Transcription and Translation, Hames & Higgins, Eds. (1984); Animal Cell Culture, Freshney, Ed. (1986); Immobilized Cells and Enzymes (IRL Press, 1986); Perbal, A Practical Guide to Molecular Cloning; Modern Methods of Organic Synthesis, W.Carutherres ain Goldham, 4th Edition, Cambridge Univertiy Press, 2004.

### Preparation of myricetin and its derivatives

In one embodiment, the native myricetin represented by formula (I) can be provided by extracting from plant as described in Chinese patent application CN 102040576 or Chinese patent application 102584766. Myricetin, its chemical name (3,5,7-Trihydroxy -2-(3,4,5-trihydroxyphenyl)-4H-1-benzopyran-4-one,CAS No:529-44-2) and its Chemical formula is C₁₅H₁₀O₈. The molecular weight is 318.24 and is a flavonoid compound. The natural myricetin was widely found in a variety of plants such as fruits, vegetables, berries, herb and the like. It is mainly extracted from leaves, peel, root of mycicacae myrica plant such as *Myrica rubra, Myrica esculenta, Myrica adenophora.* E.g. Chinese patent application CN201010293209 disclose a method of extracting myricetin from the leaves of bayberry, and Chinese patent application CN201010293209 disclose a method of exacting from *Ampelopsis grossedentata.* The myricetin purified form *Myrica* and *Vitis* plant is yellow needle-like crystalline power. The melting point is 324.0∼325.5°C, and soluble in methanol, ethanol, aceton, ethyl acetate, slightly soluble in water, insoluble in chloroform and petroleum, and is easily oxidized and become to green color when left in air.

In a preferred aspect, the natural myricetin is extracted and purified from *Myrica* plants and *Vitis* plant. Optionally, the natural myricetin represented by formula (I) below can be extracted and purified from myrica or vitis plant. Myrica plant includes but is not limited to one or more plants consisting of *Myrica rubra, Myrica esculenta, Myrica adenophora.* The vitis plant includes but not to one or more plants consisting of t *Ampelopsis cantoniensis, Ampelopsis megalophylla, Ampelopsis brevipedunculata.*

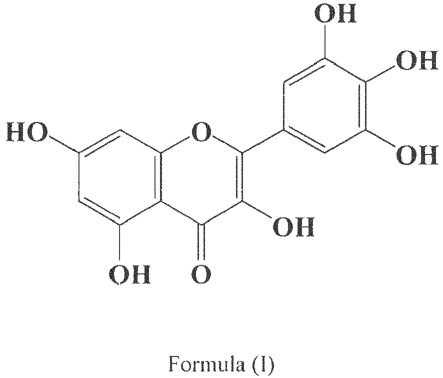

As described above, myricetin derivatives represented by formula (II) below is provided by well known organic synthesis methods. The skilled person in the art could easily select solvent, temperature, pressure and other reaction conditions suitable for the synthesis experiments. And the materials are commercially available or can be easily prepared by the well know method to the skilled person. 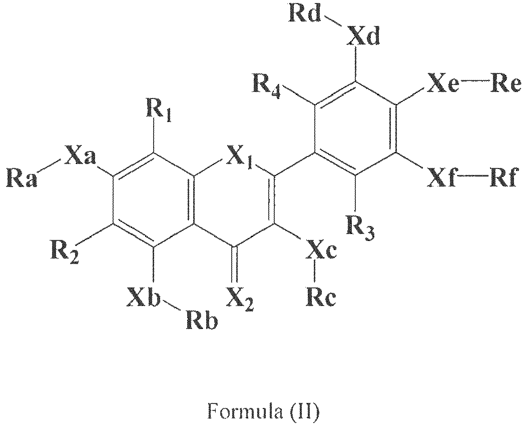 Wherein,
Xa, Xb, Xc, Xd, Xe, Xf, X₁ and X₂ is each independently selected from oxygen atom (O), sulfur atom (S); Ra, Rb, Rc, Rd, Re and Rf is each independently selected from hydrogen atom (H), (C₁-C₁₀) or (C₁-C₁₀)-O-(C₁-C₁₀) alkyl substituted by from 1 to 5 Ry, Ry is selected from Rq, or (C₂ -C₁₀) alkyl, (C₃ -C₁₀) alkyl, (C₃ -C₁₀) cycloalkylalkyl, (C₈-C₁₄) dicycloalkyl, (C₈-C₁₄)tricycloalkylalkyl, (C₅-C₁₀) cycloalkylakyl, (C₃ -C₁₀) cycloalkyl, (C₈-C₁₄) dicycloalkylalkyl, (C₈-C₁₄) tricycloalkylalkyl, phenyl, naphthyl, (C₁₄) aryl, each of them can be substituted by one or more Rz, Rz is (C₁-C₆) alkyl, (C₂ -C₆) alkenyl, (C₁ -C₆) alkynyl, (C₃ -C₈) cycloalkyl, (C₆-C₈) cycloalkenyl, phenyl, 3-5 membered heterocyclics, C(halo)₃, CH(halo) ₂.

R₁, R₂, R₃ and R₄ is each independently selected from hydrogen atom (H), (C₁-C₁₀) alkyl, (C₂-C₁₀) alkenyl, (C₂-C₁₀) alkynyl, (C₃-C₁₀) cycloalkyl, (C₈-C₁₄) dicycloalkyl, (C₈-C₁₄) tricycloalkylalkyl, phenyl, naphthyl, (C₁₄) aryl, each of them can be non-substituted, or one or more substituted Rq with one or more Rz, Rq can be CN, OH, halogen, N₃, NO₂, N(Rz)₂, =NRz, CH=NRz, NRzOH, ORz, CORz, C(O)ORz, OC(O)Rz, OC(O)ORz, SRz, S(O)Rz or S(O)₂Rz.

Preferably, Xa, Xb, Xb, Xd, Xe, Xf, X1 and X2 is each independently selected from oxygen atom (O),. Preferably, Ra, Rb, Rc , Rd, Re, Rf, R1, R2, R3 and R4 is each independently selected from hydrogen atom (H).

The term "alkyl" as used herein refers to a saturated straight or branched hydrocarbon, such as a straight groups of 1-6, 1-10, 2-10, 3-10 or 1-12 carbon atoms, referred to herein as (C₁-C₁₀) alkyl, (C₂ -C₁₀) alkyl, (C₃ -C₁₀) alkyl, (C₁ -C₁₂) alkyl, (C₁ -C₆) alkyl, respectively. Exemplary alkyl groups include, but are not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyll-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, heptyl, octyl, etc. In certain embodiments, alkyl refers to (C₁ -C₆) alkyl.

The term "cycloalkyl" as used herein refers to a monovalent saturated or unsaturated cyclic hydrocarbon group of 3-8, 3-10 carbons, referred to herein, e.g., as "C4 cycloalkyl" derived from a cycloalkane. Exemplary cycloalkyl groups include, but are not limited to, cyclopropyl, cyclobutyl, cyclopentyl, cycloneopentyl, cyclohexanyl, cycloheptyl, cyclooctyl, etc.

The term "cycloalkylalkyl" as used herein refers to a monovalent saturated or unsaturated bicyclic, bridged bicyclic, or tricyclic hydrocarbon group with a saturated straight or branched hydrocarbon of 4-10, 8-14, 5-10, referred herein, e.g., as "C6 dicycloalkylalkyl" derived from a alkylcycloalkane. Exemplary dicycloalkylalkyl groups include, but are not limited to, dicyclopropylmethyl, dicyclopropylethyl, dicyclobutylmethyl, dicyclobutylethyl, dicyclopentylmethyl, dicyclopentylethyl, dicyclohexanylmethyl, dicyclohexanylethyl. Exemplary tricycloalkylalkyl groups include, but are not limited to, tricyclopropy methyl, tricyclopropylethyl, tricyclobutylmethyl, tricyclobutylethyl.

The term "aryl" as used herein refers to a mono-, bi-, or other multicarbocyclic, aromatic ring system. In certain embodiment, aryl refers to a monocyclic and/or bicyclic, 5 to 6 membered ring system, referred herein as (C₁₄) aryl.

The term "alkenyl" as used herein refers to an unsaturated straight or branched hydrocarbon having at least one carbon-carbon double bond, such as a straight or branched group of 2-6, 2-10 carbon atoms, referred to herein as (C₂-C₆) alkenyl , (C₂-C₁₀) alkenyl, respectively. Exemplary alkenyl groups include but are not limited to, vinyl, allyl, 2- allyl, butenyl, 3- butenyl, 1-methyl-2-allyl, 2-methyl-2-allyl, pentenyl, 2-pentenyl, 3-pentenyl, 4-pentenyl, 1-methyl-2-butenyl, 2-methyl-2-butenyl, hexenyl, 2-hexenyl, 3-hexenyl, 4-hexenyl, 5-hexenyl, heptenyl, octenyl, Decenyl, butadienyl, pentadienyl, hexadienyl, 2-ethylhexenyl, 2-propyl-2-butenyl.

(C₆-C₈) cycloalkenyl as disclosed herein includes, but are not limited to cyclopentenyl, cyclohexenyl, etc.

The term "alkynyl" as used herein refers to an unsaturated straight or branched hydrocarbon having at least one cabon-cabon triple bond, such as a straight or branched group, referred herein as (C₂-C₁₀) alkynyl. Exemplary alkynyl groups include, but are not limited to, ethynyl, propynyl, 2-propynyl, butynyl, 2-butynyl, 3-butynyl, pentynyl, 2-pentynyl, 3-pentynyl, 4-pentynyl, 1-methyl-2-butynyl, 3-methyl-2-butynyl, 3,3-dimethyl-1-butynyl, 2-ethynyl-cyclopentyl, 2-ethynyl-cyclohexyl, hexynyl, methylpropynyl,etc. 3-5 membered heterocyclics as disclosed herein contain one or more hetero atoms such as S, N or O, or combination thereof. Exemplary 3-5 membered heterocyclics includes, but are not limited to, pyridine, piperidine, morpholine, pyrrole, furan, thiophene, imidazole, oxazole, thiazole, pyrazole, pyrimidine, pyrazine or pyridazine rings.

The term "halo" or "halogen" as used herein refer to F, Cl, Br, or I.

In one embodiment, the myricetin or its derivatives comprising isomers, racemic or non-racemic mixtures of the isomers, oxides, pharmaceutically acceptable salts and hydrates thereof is provided. Also the variant of myricetin and its derivatives is provided by amination, carboxylation, aldehydration, benzylation and etc., with different activity and physicochemical properties.

In one embodiment of the present invention, using potential candidate compounds, an enzyme kinetic experiments to screen an inhibitor for the collagen degradation induced by the increased cathepsin K activity is made. Particularly, it demonstrates that myricetin and its derivatives can completely inhibit cathepsin K activity induced collagen degradation in the enzyme kinetics screening experiments.

In one embodiment of inhibitory effect on collagens degradation caused by cathepsin K, the weight ratio of cathepsin K and collagen was 1:1-10000, preferably 1:10-1000, more preferably 1:10-500, and most preferably 1:20-100. When the weight ratio of cathepsin K and collagen was 1:1-10000, the weight ratio of cathepsin K and candidate compound was 1:1-10000, preferably 1:1-1000, more preferably 1:1-200, and most preferably 1:1-100 to achieve complete inhibition of cathepsin K induced collagen degradation.

In a certain embodiment, an appropriate assay in vitro or in vivo assay has been performed to determine the therapeutic effectiveness of myricetin or its derivatives, as well as the suitableness of the medication comprising them, respectively. For various therapeutic applications, the typical cell involved in subject' symptoms can be measured in vitro to determine whether the expected effects has been achieved according to the administration of a medicament comprising myricetin or its derivatives. If the subject is administered a therapeutic dose of myricetin and its derivatives disclosed herein, the treatment of the symptoms will be successful. The subject shows that, one or more observable and/or measurable symptoms will be alleviated or disappear.

In another embodiment of this invention, the prepared myricetin and its derivatives can be used for the treatment, amelioration and/or prevention of pathological symptoms or diseases caused by cathepsin K induced collagen degradation, to a subject in need thereof. Preferably, the subject is having or suffering from, or at risk of the symptoms or disease.

Optionally, the pathological symptoms or the diseases comprising one or more the pathological symptoms or the diseases selected from the group consisting of arthritis, rheumatoid spondylitis, multiple myeloma, disc injury, osteoporosis, cardiovascular disease, myocarditis, deposition of lipids caused by high cholesterol, high blood pressure, metabolic disorders, obesity, atherosclerosis, prostatitis, respiratory infectious diseases, Alzheimer's disease, diabetes, aging disease, coronary heart disease, male sexual function, loss of appetite, chronic obstructive pulmonary emphysema, hepatitis, fatigue, neurasthenia, cerebral infarction, asthma, cough, adult respiratory distress syndrome. Preferably, consisting of arthritis, rheumatoid spondylitis, multiple myeloma, disc injury, myocarditis, steoporosis, metabolic disorders, obesity, high cholesterol, atherosclerosis, high cholesterol, high blood pressure. More Preferably, the pathological symptoms or disease is arthritis or rheumatoid spondylitis.

The arthritis comprises regular joint pain, swelling, deformation, movement disorder, recurrent symptoms, and chronic pain from general sense of joints, joint soft tissue, muscle, bones. In a certain embodiment, the prepared myricetin and its derivatives can be used for treatment, amelioration and/or prevention of arthritis caused by collage degradation due to the increased cathepsin K activity in a patient need thereof, comprising one or more arthritis selected from the group consisting of arthritis, rheumatoid arthritis, rheumatoid arthritis, osteoarthritis, joint pain, osteonecrosis lumbar disc herniation, bone hyperplasia, hyperplastic spondylitis, ankylosing spondylitis, gouty arthritis, cervical spondylosis, back pain disease, disc herniation, frozen shoulder, acute and chronic synovitis, bursitis , tenosynovitis, osteoarthritis, osteoporosis, frozen shoulder, fracture sequelae, tennis below, meniscus injury, fibromyalgia, fibrositis, postpartum rheumatism, reactive arthritis, septic arthritis, traumatic arthritis , psoriatic arthritis, amyloidosis joint disease, acromegaly, enteropathy arthritis, such as systemic lupus erythematosus, Sjogren's syndrome, scleroderma, autoimmune diseases and tumor concurrent non-erosive joint inflammation.

As used herein, the terms "treat" or "treating" or "treatment" or "alleviate" or "alleviating" or "alleviation" refers to both therapeutic treatment and prophylactic or preventative measures, wherein the object is to prevent or slow down (lessen) the targeted pathologic condition or disorder. A subject is successfully "treated" for a pathological symptoms or disease if, after receiving a therapeutic amount of the myrecetin or its delivetives according to the methods described herein, the subject shows bservable and/or measurable reduction in or absence of one or more signs and symptoms of myricetin or its derivatives. It is also to be appreciated that the various modes of treatment or revention of medical conditions as described are intended to mean "substantial" or "significant" , which includes total but also less than total treatment or prevention, and wherein some biologicallyor medically relevant result is achieved.

As used herein, "prevention" or "preventing" of a disorder or condition refers to a compound that, in a statistical sample, reduces the occurrence of the symptoms or disorder in the treated sample relative to an untreated control sample, or delays the onset or reduces the severity of one or more symptoms or disorder relative to the untreated control sample.

As used in this specification and the appended claims, the singular forms "a", "an" and "the" include plural referents unless the content clearly dictates otherwise. For example, reference to "a method" includes a combination of two or more methods, and the like.

In another embodiments of the present invention, the novel pharmaceutical drug represented by formula (I) or formula (II) is used in manufacturing a medicament, food, nutraceutical or cosmetics, and composition thereof for treatment, amelioration and/or prevention of disease or pathological symptoms related to collagen degradation due to the increased cathepsin K.

In a further embodiment of the present invention, manufacturing a pharmaceutical drug comprising the compound represented by formula (I) or formula (II) in a form of oral formulation, topical formulation, inhalation formulation, nasal spray, rectal preparations, percutaneous preparations and injection preparation. Preferably, is in a form of oral formulation, topical formulation or inhalation formulation.

The term "subject" or "patient" are used interchangeably and include any animal, including mammals, preferably mice, rats, rabbits, dogs, cats, swine, cattle, cows, sheep, horses, or primates, other rodents and most preferably humans. The compounds disclosed herein can be administered to a mammal, such as a human, but can also be other mammals such as an animal in need of veterinary treatment, *e. g*., domestic animals *(e. g*., dogs, cats, and the like), farm animals *(e. g*., cows, sheep, pigs, horses, and the like) and laboratory animals *(e. g*., rats, mice, guinea pigs, and the like). The mammal treated in the methods of the invention is desirably a mammal with the collagen degradation by the increased cathepsin K activity. Most preferable is or are human(s) suffering from or at the risk of collagen degradation by the increased cathepsin K activity.

In one embodiment, a suitable animal model systems can be used for testing candidate therapeutic compounds. Those animal model systems include, but are not limited to, e.g. non-human primates (e.g. baboons, chimpanzees, monkey), pet animals (e.g. cats, dogs, snakes, etc.), farm animals (e.g. pits, horses, cattle, goats, etc.), laboratory animals (e.g. rats, mice, monkeys, rabbits, etc.). Preferably, the subject is human. The disclosure is directed at least in part to treating, ameliorating and/or preventing the symptoms or disease using a disclosed compound. For example, methods of treating arthritis is provided, wherein a disclosed compound (or a composition contains the disclosed compound) is administered to a subject in need thereof, e.g. orally administered.

In some embodiments, a method of treating arthritis is provided, wherein food, nutraceutical or cosmetics comprising the compound represented by formula (I) or formula (II) is administered to a human subject in need thereof, e.g. orally.

In a further embodiment, a method of treating arthritis in a patient suffering from (or having or anticipating suffering from) or at risk of arthritis is provided, comprising administering an effective amount of the disclosed compound (or a composition includes a disclosed compound). Contemplated methods include those that slowdown (ameliorate) or prevent arthritis, as compared to, for example the subject without contemplated treatment. Also as used herein, the term "effective amount" refers to a quantity sufficient to achieve a desired therapeutic and/or prophylactic effect, e.g. , an amount which results in the prevention of, or a decrease in, the symptoms associated with an arthritis condition. The amount of a composition administered to the subject will depend on the type and severity of the disease and on the characteristics of the individual, such as general health, age, sex, body weight and tolerance to drugs. It will also depend on the degree, severity and type of disease.

The skilled artisan will be able to determine appropriate dosages depending on these and other factors. The compositions can also be administered in combination with one or more additional therapeutic compounds. Generally, a therapeutically effective amount of active component will be in the range of from 0.1 mg/kg to 10000mg/kg body weight, such that, optionally, the compound disclosed herein is prepared, and the daily dose was from 0.1 mg/kg to 10000 mg/kg body weight, preferably from about 1 mg/kg to 1000 mg/kg body weight, more preferably more preferably 1mg / kg to about 500mg/kg, and most preferably 1mg / kg to about 200mg/kg body weight. The administration intervals can be irregular and dosing frequency can vary, depending on factors described below, exemplary dosing frequency are once per day, once per week and once every two or three weeks. In some treating cases, the dosing frequency can be increased from one to six times per day. The skilled person in the art might appreciate that the dosages including the best amount and a single administration interval for use in any application will depend on the factors, such as nature and extent of the conditions or disease being treated, not only on the relative biological efficacy of compound or composition selected, but also with the route and positions of administration, the formulation of compounds or compositions, varying from patient to patient, e.g. age, health status, and etc. The most preferred regime can be made up according to the conventional technology. Also it should be understood that the best process of the treatment, which is in the given number of days, the daily dose of the compound or the composition per day may be determined by skilled person using a conventional treatment tests or assays. In therapeutic applications, a relatively high dose in a relatively short time interval is sometimes necessary until the disease process is suppressed or terminated, and preferably until the treated target subject shows partial or complete alimentation of the disease or symptoms. Thus, the patient can be administered in a prophylactic regime. The skilled person in the art can recognize that a certain factors can affect the effective dose and time of the treated subject, including but are not limited to, the severity of the disease or disorder, previous treatments, health and / or subjects age and the presence of other diseases. Moreover, the described therapeutically effective amount of a therapeutic composition for the treated target may include a single treatment or series of treatments.

For treating clinical conditions and diseases noted above, the medicament of this invention may be administered orally, topically, parenterally, by inhalation spray or rectally in dosage unit formulations containing pharmaceutically acceptable carriers, adjuvants and vehicles. The term "parenteral administration" as used herein includes subcutaneous injection, intravenous, intramuscular, intrasternal injection or infusion techniques.

The compounds of the present invention may be administered with at least one another compound together. For example, it can be used with the commonly used compounds for the treatment of arthritis including, but is not limited to, salicylic acid preparations, ibuprofen, Fenbid, indomethacin, Glucosamine indomethacin, sulindac (pivot Lida), Rantudil, naproxen, celecoxib, rofecoxib, sodium hyaluronate (Altz, Hyalgan, Sofastin), nimesulide, indomethacin, aspirin, feldene, flufenamic acid, gold salt, penicillamine, chloroquine and its derivatives, levamisole, adrenal corticosteroids (e.g. prednisone), cyclosporine, sulfasalazine, cyclophosphamide, sulfur azathioprine, methotrexate, y interferon, specific antibodies (McAb), captopril, inorganic or organic calcium preparation such as calcium chloride, calcium carbonate, , zinc preparation such as zinc sulfate, chloroformate dual disodium phosphate, hydroxyethyl sodium diphosphate, isopropoxide flavonoid, D-glucosamine hydrochloride, chondroitin sulfate, soy isoflavones. It should appreciate that the skilled person in the art can make selections based on the disease or pathological symptoms to administer the compounds of the present invention in combination with the compounds mentioned above.

The compounds of the present invention can be administered with at least one herb. The herb comprises one or more herb from the group consisting of papaya pills, large meridian- activating pill, small meridian-activating pill , bupleurum, angelica, salvia, cassia twig, peony, orange peel, pinellia, speranskia, pterygium, rehmanniae, bellflower, cyperus, mustard seed , aconitum , honeysuckle, gentiana, mulberry, poria. It should appreciate that the skilled person in the art can make selections based on the disease or pathological symptoms to administer the compounds of the present invention in combination with the herbs mentioned above.

The compounds or herbs as a multiple agent described above can be manufactured in an individual formulation, or in combination with the compound or its derivatives thereof as disclosed herein to form a composition formulation. In any case, the multiple therapeutic agents may be administered in any order or even sequentially or simultaneously. If simultaneously, the multiple therapeutic agents may be provided in a single, unified form, or in multiple forms (by way of example only, either as a single tablet, or each independently separate tablets or capsules of the multiple agents). One of the therapeutic agents may be given in multiple doses, or both may be given as multiple doses. If not simultaneous, the timing between the multiple doses may vary from more than zero weeks to less than about one weeks, less than about two weeks, less than about four weeks, less than about 2 months, less than about 4 months, or less than about one year. As used herein, the term "simultaneous" therapeutic use refers to the administration of at least two active ingredients by the same route and at the same time or at substantially the same time.

As used herein, the term "separate" therapeutic use refers to an administration of at least two active ingredients at the same time or at substantially the same time by different routes.

As used herein, the term "sequential" therapeutic use refers to administration of at least two active ingredients at different times, the administration route being identical or different. More particularly, sequential use refers to the whole administration of one of the active ingredients before administration of the other or others commences. It is thus possible to administer one of the active ingredients over several minutes, hours, or days before administering the other active ingredient or ingredients. There is no simultaneous treatment in this case.

In one embodiment of the invention, the pharmaceutical compounds or compositions comprising at least one active ingredient, myricetin or its derivatives or a pharmaceutically acceptable salt thereof in association with a pharmaceutically acceptable excipient such as a carrier or dilution were provided up in a form suitable for oral, topical, inhaled, nasal, rectal, percutaneous or injectable administration. The composition suitable for oral can be prepared into a variety of dosage forms, for example, tablets, powders, granules, lozenge, effervescent tablets, syrup, emulsion, controlled release preparations, rapid-dissolving preparations, oral liquid dosage form. Composition suitable for topical administration, for example may be smear-type formulations, creams, topical ointment, lotion, topical liquid, aerosol sprays or gas spray. Composition suitable for inhalation may be, for example, solutions, dispersions, powder, etc. It is also possible to extract myricetin compound from Myrica plants and grape plants with high purity to prepare liposome wrapped nano myricetin, or prepare injectable sustained release formulations. For the mentioned pharmaceutical compositions comprise 0.001% to 99% , preferably 0.01% -90% by weight, more preferably 10% -80 % by weight, and most preferably 25-70% weight of the active ingredient, respectively,

For pharmaceutical formulation of the present invention, for example, an oral formulation, the use of pharmaceutically acceptable excipients include, but are not limited to, a lubricant such as magnesium stearate, calcium stearate or zinc stearate, ocosanoic acid glycerol ester, sodium stearyl fumarate, talc, silica gel, the disintegrating agents such as starch, cyclodextrin, carboxymethyl cellulose, cross-linked carboxymethyl cellulose, cross-linked polyvinyl pyrrolidone, the dilution agents or compression agents, such as lactose, sucrose, mannitol, xylitol, erythritol, sorbitol, microcrystalline cellulose, and flavoring ingredients or others, such as strawberry, orange, banana, mint, honey.

The carrier particles may be lactose or sucrose crystals or spheres, or composite spheres or particles, such as by using the starch as a binder to form the granular sucrose or sugar spheres, using starch as a binder to form calcium carbonate spherem spheres or maltodextrin. Carrier particles can also be any other pharmaceutical acceptable excipient particles, such as hydroxypropyl cellulose particles, guar gum particles, xanthan gum particles. Carrier may have many forms, while for all of which, the choices and amount adjustment are clearly within the capabilities of the skilled artcian by the oral administration, the compounds or the compositions of myricetin or derivative comprising them dissolved in a liquid food or health products, such as optionally dissolved in an flavored aqueous liquid or water-alcohol solution. It may be incorporated into a solid swallowable excipient, for example, in the form of granules, pills, tablets, coated tablets. It may also be placed in the liquid of food or health products, optionally in swallowable capsules. In a certain embodiment for multiple oral compositions, in particular, it can be food additives for oral administrated embodiments. Conventionally manufacturing methods can be used for formulation of enteric-coated tablets, gel capsules, gels, emulsions, tablets, capsules or solutions. In particular, according to the present invention, the active agent may be incorporated into dietary supplements, fortified foods or any other form of health care products, such as food or health products, or being compressed or uncompressed in powder. These powders can be diluted with water in the soda, dairy products or soy derivatives, or can be incorporated into a food or health products.

In another embodiment of the present invention, cosmetics comprising myricetin or its derivatives is manufactured. The cosmetic may be prepared as cream type of pharmaceutical formulations, topical ointment or plaster, topical solutions, dispersions, dry powder, dry liquid sprays, gas sprays or a variety of formulation suitable for injection. By injection or topical administration to a subject, it can be expected to treat and / or prevent pathological condition or disease caused by collagen degradation due to the increased human cathepsin K activity.

The following examples are for a better understanding of the present invention, but the embodiments are not limited to those examples.

### Examples

### Example 1. Myricetin suppresses cathepsin K induced collagen degradation

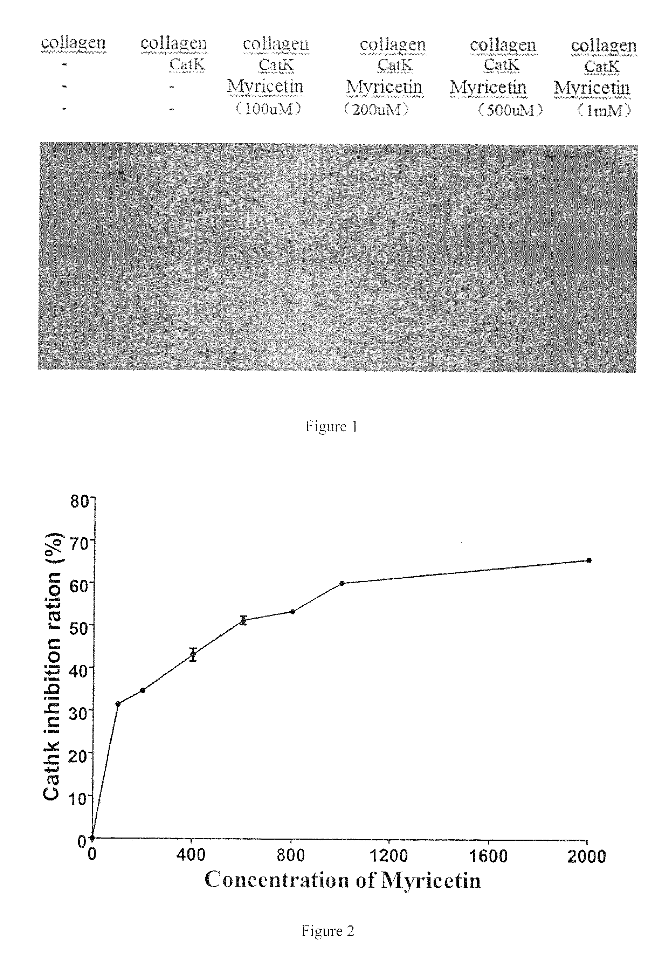

Firstly, following the method reported by Linnevers et al., the genetic engineering techniques were used to express the inactive precursor protein of human cathepsin K in yeast cells from encoded region of human cathepsin K. After purification, the protein was digested by pepsin to remove the precursor inhibitor peptide of the cathepsin K, and to prepare the active cathepsin K for screening the inhibitor compounds (Linnevers et al., Protein Sci. 1997 Apr; 6 (4) :919-21). A series of Chinese herbal medicine of monomer myricetin were purchased from Shanghai Jingke Chemical Co., Ltd.,(Cat. No. A0145, 3,5,7,3 ', 4', 5'-hexahydroxyflavone, CAS No :529-44-2). It was purified from natural plant (the purity is more than 98.5% confirmed by NMR and HPLC purity assay) and was used as an inhibitor candidate compound. Meanwhile, collagen type I (Cat No. SU2087) and collagen type II (Cat No. SU2088) were purchased from SIGMA and were used as a substrate for cathepsin K. For screening experiments, 100 mg collagen and 1 ug cathepsin K were added into 50 mM phosphate buffer (pH6.0), then myricetin compounds dissolved in 20% ethanol were added with the final concentrations of 100,200,500,1000 uM, respectively, finally an additional 50 mM of phosphate buffer (pH 6.0) was added again to bring a total volume of 500 uL. The reactions were incubated at 37 °C for 15 hours in a water bath. As a positive control without any candidate compound, 100 ug collagen, 1 mg cathepsin K, and 50 mM phosphate buffer solution (pH6.0) were added to bring a total volume of 500 uL. As a negative control without cathepsin K, 100 ug collagen, 1000 uM candidate compounds, and 50 mM phosphate buffer solution (pH6.0) were added to bring a total volume of 500 uL. After completion of the reactions, the reaction samples were applied to 10% SDS-PAGE gel electrophoresis, coomassie blue staining and destaining, and the intensity of the stained protein bands were analyzed. The experiments were repeated for 5 times to verify the results. After a series of screening, the effective cathepsin K inhibitor was obtained. The inhibitor is myricetin. 100 uM myricetin is able to achieve the complete inhibition of the cathepsin K activity (see Figure 1).

### Example 2. Myricetin inhibits cathepsin K activity

In order to determine the inhibitory effect of myricetin on cathepsin K activity, a cathepsin K specific fluorogenic substrate HD-Ala-Leu-Lys-AMC (Cat. No. 104881-72-3, purchased from GL Biochem (Shanghai) Co., Ltd.) was used and Cary Eclipse fluorescence spectrophotometer (Agilent Technologies Inc., Beijing) for quantitative analysis was applied. 1 ug of cathepsin K-specific fluorogenic substrate HD-ALK-AMC,1 ug cathepsin K, myricetin compounds dissolved in 20% ethanol with final concentrations of 100,200,500,1000 uM were added into 50 mM of phosphate buffer. An additional 50 mM of phosphate buffer (pH6.0) was added to bring a total volume of 500 uL. And the reaction sample was inhibited at 37°C for 15 hours in a water bath. As a positive control without any candidate compound, 1 ug of cathepsin K specific fluorogenic substrate HD-ALK-AMC and 1 ug of cathepsin K, and 50 mmol phosphate buffer solution (pH6.0) were added to bring a total volume of 500 uL. As a negative control without cathepsin K, 1 ug of cathepsin K specific fluorogenic substrate HD-ALK-AMC, myricetin compounds dissolved in 20% ethanol with final concentrations of 100,200,500,1000 uM were added into 50 mM of phosphate buffer. An additional 50 mM of phosphate buffer (pH6.0) was added to bring a total volume of 500 uL. After completion of the reaction, the samples were measured at 390 nm excitation wavelength and 460 nm emission wavelength for fluorescence intensity changes assay, and the value of cathepsin K activity was calculated. As shown in Figure 2, myricetin shows significant inhibitory effect on cathepsin K activity, and the effect is enhanced as the concentration of myricetin increased (see Figure 2).

In conclusion, myricetin has remarkable inhibitory effect on cathepsin K, and suppresses collagen degradation by cathepsin K activity. It can be expected that myricetin can prevent, ameliorate and/or treat pathological symptoms and/or disease associated with cathepsin K induced collagen degradation.

### Example 3. Animal safety evaluation on myricetin

### 3.1 Experimental animal species and characteristics

BALB / C mice were bred by Harbin Veterinary Research Institute of Experimental Animal Center, Chinese Academy of Agricultural Sciences, and the animal quality permit number is Heilongjiang-SYXK 2006-032. The selection conditions are age at 9 weeks to 10 weeks, weighing about 20 g, half male and half female.

### 3.2 Feeding conditions

The animal laboratory had regular air ventilation, good lighting, and constant laboratory room temperature. There were five mice per cage, and they were fed with extruded feed produced specifically for mice, with free access to drinking water. Before the experiment started, the animals were observed for a week of eating, activity and the manure, and only the healthy mice were selected for further experiments.

Firstly, the acute toxicity tests were conducted on to evaluate the safety of myricetin in order to provide the basis for the therapeutic application of myricetin. In this study, the maximum tolerated dose acute toxicity test was used. Twenty of BALB/C mice with the healthy body weight about 20 g, half male and half female were selected, The clinical intended route of administration is oral, so this experiment used intragastric administration. Myricetin (purchased from Shanghai Chemical Co., Ltd. Item No. A0145, Myricetin; 3,5,7,3,4', 5'-hexahydroxyflavone, CAS No :529-44-2. Pure and natural extracts, purity is more than 98.5% confirmed by NMR and HPLC purity analysis) was modulated into a stick-like with distilled water. Dosed at 100 g / kg body weight, gavage was conducted to the mice (about 200 mg myricetin per mouse). Before the gavage, the animals were fasted overnight with free access to water. After the administration, the mice had a normal diet, and were observed for symptoms of poisoning and death, and were weighed weekly for a period of 2 weeks. Under the same conditions, there was a control group of the same batch that was fed with the normal food without myricetin under the observation. Experimental results showed that all mice were free of any symptoms of poisoning, which proved myricetin is a non-toxic substance.

### Example 4 The effects of myricetin treatment on type II collagen-induced arthritis in mice

In order to verify the therapeutic application of myricetin and its derivatives in pharmaceuticals, food, health and cosmetics products for the prevention and/or treatment of cathepsin K activity induced collagen tissue degradation that leads to arthritis, osteoporosis, disc injury, myocarditis, and metabolic disorder caused lipid deposition, obesity, hyperlipidemia, atherosclerosis, hypertension, we used type II collagen transcutaneous immunized mice to prove the treatment effect of myricetin against arthritis mice that induced by type II collagen, and further studied myricetin's performance of treatment and preventive effects on the clinical disease by suppressing cathepsin K activity.

### 4.1 The construction of type II collagen-induced arthritis mice model

Firstly, same strains and sources of animals as shown in Example 2 were selected, and total 105 of 9-week-old BALB / C mice were fed under the same conditions. There were 70 mice that each had subcutaneous injection of 200 ug of Freund's adjuvant emulsified bovine type II collagen. 15 days after the first immunization, subcutaneous injection was applied again at 200 ug per mouse with incomplete Freund's adjuvant emulsified bovine type II collagen to strengthen the immunity. After 24 days of the first injection, the 70 mice were divided into two groups, of which 35 mice were treated with myricetin intake orally 25 mg / kg body weight daily as a treated-group, while the other 35 were the model control group fed normally without myricetin. The remaining 35 mice were the normal control group without immunization and fed with normal food, used as a blank control.

### 4.2. Observation, evaluation and examination

### 4.2.1 Observation and Evaluation

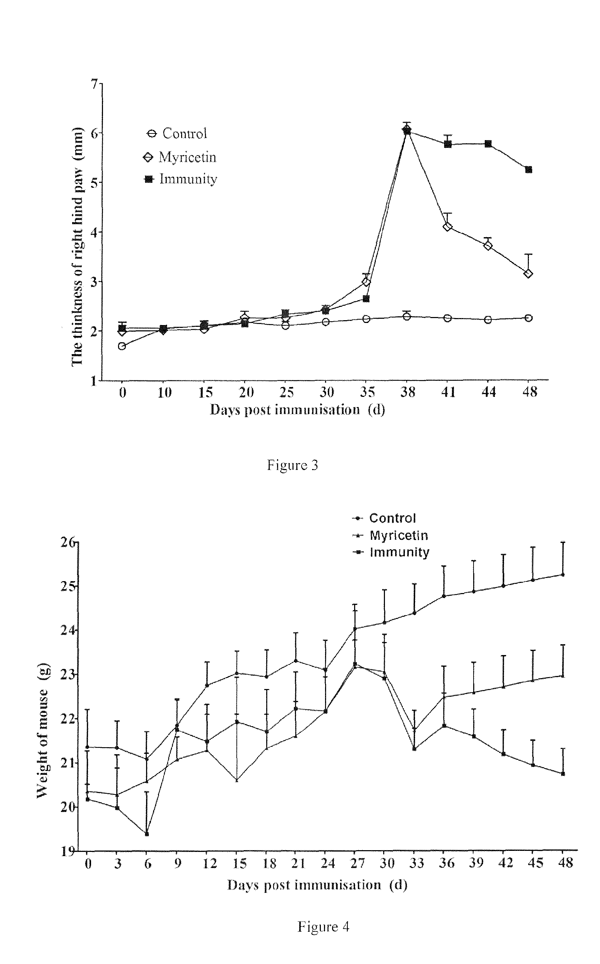

Throughout the testing period, arthritis conditions of the mice and arthritic conditions after the treatment were observed. After immunization with type II collagen on mice, the conventional methods were used by measuring the degree of paw swelling (thickness) (see Figure 3) and weight change (see Figure 4), and myricetin therapeutic effects were monitored and evaluated throughout the procedure (0.5 mm increased thickness for one magnitude) (see Figure 5). As can be seen from Figure 3 and 5, the immunized mice showed swollen joints since the second day of immunization (i.e. 16 days), became more obvious on the 21 days, and then gradually exacerbated, while the symptoms of the treatment group were significantly improved. The experiments were observed for 48 days in total.

On the 48 day, the blood from the heart was extracted, and the serum was separated, meanwhile all mice were killed. The mouse urinary deoxypyridinoline (urinary deoxypyridinoline) (see Figure 6), type I collagen C telopeptide (CTX- I) (see Figure 7) and serum cartilage oligomeric matrix protein (COMP) were examined, and histological treatment evaluation were conducted (see Figure 8).

### 4.2.2 Detection of the indicators in mouse serum

### 4.2.2.1 examination of urinary deoxypyridinoline in mice serum

The mouse urinary deoxypyridinoline/deoxypyridinoline kit (DPD kit from Shanghai Huyu Biotech Co., Ltd., No. HY-E3170B) was used. According to the instructions provided, iMark-BIO-RAD microplate reader (U.S. BIO-RAD) was used to detect the concentration of mouse urinary deoxypyridinoline at 450 wavelength (see Figure 6). The results showed the concentration of mice serum urinary deoxypyridinoline from the treatment group was significantly lower than that of the control group (P <0.001), which indicated arthritis in mice after the treatment had been significantly improved.

### 4.2.2.2 The content detection of type I collagen C-terminal peptide in mice serum

Rat C-terminal peptide of type I collagen (CTX-I) ELISA Kit from Shanghai Jinma Biotech Co., Ltd., NO Wk30026) was used. According to the method provided by the kit instruction, the change in the content of type I collagen C-terminal peptide in mice serum was detected to evaluate the effect that myricetin prevented type I collagen degradation by suppressing cathepsin K (see Figure 7). The results showed in the treatment control group, the content of type I collagen C-terminal peptide was significantly lower than that of the model control group (P <0.001), and even close to the normal control group, suggesting that myricetin can significantly inhibit type I collagen degradation in mice serum.

### 4.2.2.3 The content detection of cartilage oligomeric matrix protein in mice serum

The cartilage oligomeric matrix protein ELISA kit (from Shanghai Yihan Biotech Co., Ltd., No. SU10580) was used. According to the methods provided by the kit's instructions, the change in the content of cartilage oligomeric matrix protein in mice serum was detected to evaluate the effect that myricetin prevented the content change ofcartilage oligomeric matrix protein induced by type II collagen degradation by suppressing cathepsin K. The results showed in the treatment control group, the content of cartilage oligomeric matrix protein was significantly lower than that of the model control group (P <0.005) (see Figure 8), suggesting that myricetin can significantly inhibit the loss of cartilage oligomeric matrix protein in mouse serum, and thus protect the joint cartilage.

### 4.2.3 Mice joint histological observation

On the 48 days of the experiment, blood was extracted from the hearts , from which the serum was separated, and all mice were killed.

After killing of the mice, the paws were fixed with 4% formaldehyde for 24 hours, then decalcificated using 500 mmol of EDTA (pH7.4). The paraffin sections were prepared using the conventional method and stained with hematoxylin / eosin staining method. Under the microscope, comparing with the normal control group, the immunized control group had gradually increased synovial surface lesions, gradually increased cellulose-like substance and synovial cells, gradually thickened synovium and articular capsule, edema, degeneration, and coarse fibrosis. The synovial surface almost disappeared with a rough surface in a grid pattern, and there were a large number of macrophages. Yet for the Myricetin treated mice, the synovial surface had significantly reduced pathological changes, and quite similar to that of the normal control group. It can be concluded that by myricetin therapeutic administration, the mice arthritis is mostly recovered (see Figure 9).

### 4.2.4 The comprehensive evaluation of the effect of myricetin on joint arthritis in mice

Based on mice joint tissue sections, according to methods from Larsson et. al. (Rheumatology (Oxford), 2004, 43 (4),428-434),the therapeutic effect of myricetin on rheumatoid arthritis in mice was evaluated, as well as the degree of arthritis. Main evaluation were based on: (1) the size of the joint cavity, inflammatory cell infiltration, cartilage damage and fibrin deposition. From three individual evaluations, it was divided into three levels, which are 0 for normal, level 1 for the light degree of arthritis, level 2 for the moderate arthritis and level 3 for severe arthritis (see Figures 3 and 5). (2) The weight change (see Figure 4). (3) histology examination (see Figure 9) and evaluation (see Figure 10 and 11). It showed that myricetin can significantly improve the pathological symptoms of arthritis, and play a role in treating rheumatoid arthritis. The above results showed that: compared with the model group, the control group under the myricetin treatment significantly reduced the degree of arthritis (P <0.05), which means myricetin was very effective compound for the treatment of rheumatoid arthritis. In conclusion, myricetin can significantly reduce the loss of collagen as well as the lose of the cartilage oligomeric matrix protein (COMP), and significantly reduce the arthritis symptom. The animal experiments, for example, the treatment on type II collagen induced arthritis in mice, demonstrated that myricetin inhibits cathepsin K induced collagen degradation in vivo. It can be derived that myricetin prevents, ameliorates and/or treats osteoporosis, arthritis, disc injury, myocarditis, metabolic disorders caused by the deposition of lipids, obesity, hyperlipidemia, arteriosclerosis, hypertension, effectively caused by cathepsin K activity induced collagen degradation.

The beneficial effects of the present invention are: myricetin can inhibit cathepsin K activity and suppress collagen degradation, and thus can be used to prevent disease that caused by cathepsin K activity induced collagen degradation and develop related therapeutic medicament.

The invention is not limited to the particular embodiments described herein, which is a single description as a separate aspect of the present invention. A skilled person in the art might understand that the present application can be modified and changed if not departing from the spirit and scope of the application. According to the above descriptions, besides those examples, it is obvious to a skilled person in the art that there are other applications within the same scope or equivalent to those mentioned in the present invention. Such changes and modifications intend to fall within the scope of the appended claims. The disclosure is limited only by the appended claims and claims with such an equivalent scope to the full limit. It should be understood that the present disclosure is not limited to the specific methods, reagents, compositions and biological systems, and assuredly, the method, reagents, compositions and biological systems may vary. It should be also understood the terminology herein is only used to describe the particular embodiments, and not to be restrictive.

In addition, while the disclosure is described using Markush group features and aspects, the technician in this field should appreciate that the presented disclosure is described in the manner of individual or sub-group of Markush group.

The referenced or quoted patents, patent application, earlier application and publication were quoted fully in the present application, including all the attached pictures and tables, so that they will not contradict with the explicit instruction of the invention. Other embodiments could be suggested within the scope of the claims.

## Claims

1. Myricetin or derivatives thereof for use in the prevention, amelioration and/or treatment of pathological symptoms or disease caused by collagen degradation due to the human cathepsin K activity rising.

2. Myricetin or derivatives thereof for use according to claim 1 wherein the Myricetin or derivatives thereof is present in pharmaceuticals, foods, nutraceuticals or cosmetics.

3. Myricetin or derivatives thereof for use according to claim 1 or claim 2, wherein the pathological symptom or the diseases are selected from the group consisting of arthritis, rheumatoid spondylitis, multiple myeloma, disc injury, osteoporosis, cardiovascular disease, myocarditis, deposition of lipids caused by high cholesterol, high blood pressure, metabolic disorders, obesity, atherosclerosis, prostatitis, respiratory infectious diseases, alzheimer's disease, diabetes, aging disease, coronary heart disease, male sexual function, loss of appetite, chronic obstructive pulmonary emphysema, hepatitis, fatigue, neurasthenia, cerebral infarction, asthma, cough, adult respiratory distress syndrome.

4. Myricetin or derivatives thereof for use according to any one of claims 1-3, wherein the pathological symptom or disease is arthritis or rheumatoid spondylitis.

5. Myricetin or derivatives thereof for use according to any one of claim 1-4, wherein the myricetin or derivatives thereof is represented by the following formula (I) or (II), comprising isomers, racemic or non-racemic mixtures of the isomers, oxides, pharmaceutically acceptable salts and hydrates thereof: 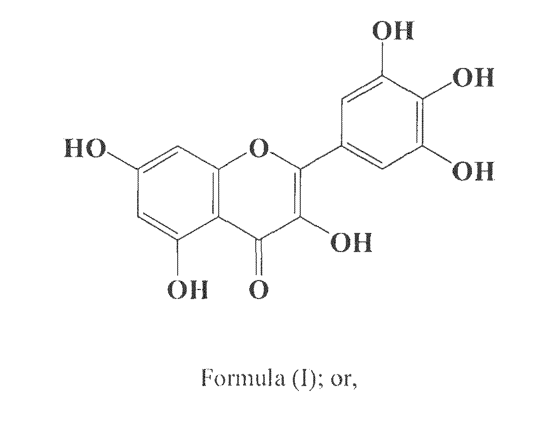 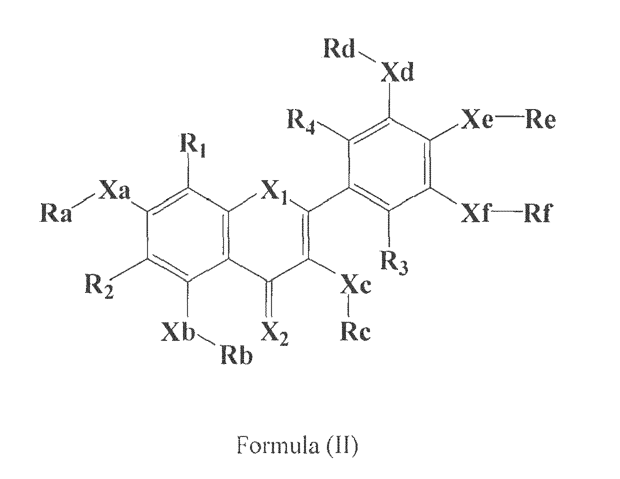 wherein
Xa, Xb, Xc, Xd, Xe, Xf, X₁ and X₂ is each independently selected from oxygen atom (O) or sulfur atom (S);
Ra, Rb, Rc, Rd, Re and Rf is each independently selected from hydrogen atom (H), (C₁-C₁₀) or (C₁-C₁₀)-O-(C₁-C₁₀) alkyl substituted by from 1 to 5 Ry, Ry is selected from Rq, or (C₂ -C₁₀) alkyl, (C₃ -C₁₀) akyl, (C₃ -C₁₀) cycloalkylalkyl, (C₈-C₁₄) dicycloalkyl, (C₈-C₁₄)tricycloalkylalkyl, (C₅-C₁₀) cycloalkylalkyl, (C₃ -C₁₀) cycloalkyl, (C₈-C₁₄) dicycloalkylalkyl, (C₈-C₁₄) tricycloalkylalkyl, phenyl, naphthyl, (C₁₄) aryl, each of them can be substituted by one or more Rz, Rz is (C₁-C₆) alkyl, (C₂ -C₆) alkenyl, (C₁ -C₆) alkynyl, (C₃ -C₈) cycloalkyl, (C₆-C₈) cycloalkenyl, phenyl, 3- to 5-membered heterocyclics, C(halo) ₃ or CH(halo) ₂;
R₁, R₂, R₃ and R₄ is each independently selected from hydrogen atom (H), or (C₁-C₁₀) alkyl, (C₂-C₁₀) alkenyl, (C₂-C₁₀) alkynyl, (C₃-C₁₀) cycloalkyl, (C₈-C₁₄) dicycloalkyl and (C₈-C₁₄) tricycloalkylalkyl, phenyl, naphthyl, (C₁₄) aryl, each of them can be non-substituted, or one or more substituted Rq with one or more Rz, Rq can be CN, OH, halogen, N₃, NO₂, N(Rz)₂, =NRz, CH=NRz, NRzOH, ORz, CORz, C(O)ORz, OC(O)Rz, OC(O)ORz, SRz, S(O)Rz or S(O)₂Rz.

6. Myricetin or derivatives thereof for use according to any one of claim 2 to 5, wherein the pharmaceuticals, foods, nutraceuticals and cosmetics are in the form of oral formulation, topical formulation, inhalation formulation, nasal spray, rectal preparations, percutaneous preparations or injection preparation.

7. Myricetin or derivatives thereof for use according to claim 6, wherein the pharmaceuticals, foods, nutraceutics or cosmetics are in the form of oral formulation.

8. Myricetin or derivatives thereof for use according to any one of claims 1 to 7, wherein the use comprises administering a pharmaceutically effective amount of the myricetin or derivatives thereof to a patient in need thereof suffering from or at risk of pathology symptoms or disease associated with collagen degradation by human cathepsin K activity rising.

9. Myricetin or derivatives thereof for use according to claim 8, the effective amount comprising administering about 0.1 mg/kg to 1000 mg/kg body weight per day to a patient in need thereof.

10. Myricetin or derivatives thereof for use according claim 5, the myricetin or derivatives thereof being in a composition comprising a pharmaceutically acceptable diluent, excipient or carrier, the amount of the pharmaceutical composition of myricetin or derivatives thereof is about 10% - 80% (w/w) of the total preparation.
